# EUROPEAN PATENT SPECIFICATION

(11) **EP 1 263 325 B1**
(45) Date of publication and mention of the grant of the patent: **01.07.2009**
(21) Application number: 01913306.5
(22) Date of filing: 05.03.2001
(51) Int. Cl.: A61B 8/00, A61K 9/14, A61K 49/00

(54) **EMBOLIC AGENTS VISIBLE UNDER ULTRASOUND**
UNTER ULTRALSCHALL SICHTBARE EMBOLISIERENDE SUBSTANZEN
AGENTS EMBOLIQUES VISIBLES PAR ULTRASONS

(30) Priority: 06.03.2000 US 519263
(43) Date of publication of application: 11.12.2002
(73) Proprietor: Boston Scientific Limited, Barbados, West Indies (BB)
(72) Inventor: MANGIN, Stephan, Ashland, MA 01721 (US)
(74) Representative: Peterreins, Frank
(86) International application number: PCT/US2001/006981
(87) International publication number: WO 2001/066016

(56) References cited:
- WO-A-98/47532
- US-A- 5 559 266
- US-A- 6 099 864
- SCHWARZ K Q ET AL: "The acoustic filter: an ultrasonic blood filter for the heart-lung machine." THE JOURNAL OF THORACIC AND CARDIOVASCULAR SURGERY. UNITED STATES DEC 1992, vol. 104, no. 6, December 1992 (1992-12), pages 1647-1653, XP009025627 ISSN: 0022-5223
- MARKUS HUGH S ET AL: "Experimental aspects of high-intensity transient signals in the detection of emboli" JOURNAL OF CLINICAL ULTRASOUND, vol. 23, no. 2, 1995, pages 81-87, XP009025622 ISSN: 0091-2751

## Description

### 1. FIELD OF THE INVENTION

The present invention relates generally to medicine. In particular, the invention is directed to embolic particles that are visible under ultrasound and which are used for embolization and occlusion in the circulatory system or duct system.

### 2. BACKGROUND OF THE INVENTION

Many clinical situations would benefit from therapeutic regulation of the vascular, lymphatic or duct system by restricting the flow of body fluid or secretions to or from organs or parts of an organ. The technique of embolization involves the therapeutic introduction of various substances into the circulation to occlude vessels so as to either arrest or prevent hemorrhaging or to defunctionalize a structure or organ. Since the 1960's, embolization has been used to treat bleeding ulcers, tumors, and trauma. It has also been used to stop hemorrhaging which can sometimes occur after childbirth.

In general, the embolization procedure involves first the giving of local anesthesia over a common artery. The artery is then percutaneously punctured and a catheter is inserted and fluoroscopically guided into the area of interest. An angiogram is then performed by injecting contrast agent through the catheter, thereby revealing the abnormality in the area. An embolic or sclerosing agent is then deposited through the catheter. The embolic or sclerosing agent is chosen on the basis of the size of the vessel to be occluded, the desired duration of occlusion, and the type of abnormality to be treated. A follow-up angiogram is usually performed to determine the specificity and completeness of the arterial occlusion. For a review of this procedure, see Feldman et al., 1983, Radiology 147:27-30; White et al., 1984, AJR 142:27-30.

Permanent or temporary occlusion of blood vessels is essential for managing various disorders such as vascular lesions, aneurysms, and arteriovenous fistulas or malformations. An aneurysm is an abnormal, balloon-like bulging of an arterial wall. Aneurysms may damage arteries that supply blood to the brain, which may lead to a stroke. Neural aneurysms, which occur nearly 30,000 times a year in the U.S., result in a subarachnoid hemorrhage leading to a number of complications.

Another example of a disorder that may be treated by embolization is varicocele. Varicocele is an abnormal dilatation of the veins of the pampiniform plexus which has been observed in 35% of men with primary infertility and in up to 80% of men with secondary infertility. While the etiology of varicocele is unclear, the goal of treating varicocele is to obstruct the refluxing venous drainage to the testis while maintaining arterial inflow and lymphatic drainage.

In addition, controlled, selective obliteration of the blood supply to tumors (or cancerous tissues) is effective in treating solid tumors such as renal carcinoma, bone tumor, uterine fibroid, and liver cancer. The theory behind this treatment is that preferential blood flow toward a tumor will carry embolization agent to the tumor thereby blocking the flow of blood which supplies nutrients to the tumor, thus, causing it to shrink.

Fibroids, also known as leiomyoma, leiomyomata or fibromyoma, are the most common benign tumors of the uterus. Arising from the muscular wall of the uterus, their origin is thought to be the muscle in the walls of uterine blood vessels. These non-cancerous growths are present in 20-40% of women over the age of 35. Fibroids are estimated to result in 200,000 hysterectomies every year. In most cases, multiple fibroids are present, often up to 50 or more. In some women, fibroids can become enlarged and cause excessive bleeding and pain; and in pregnancy, pre-existing fibroids can increase 3-5 times in size, possibly due in part to the high level of estrogen and other factors present during pregnancy. Artery ligation is not an effective treatment for uterine fibroids because blood flow can be diverted to other lateral arteries to carry nutrients to the fibroids.

While the classic treatment of symptomatic fibroids has been the surgical removal of the fibroids (myomectomy) or removal of the uterus (hysterectomy), recent advances in uterine embolization now offer a nonsurgical treatment.

Uterine embolization is aimed at starving fibroids of nutrients. Numerous branches of the uterine artery may supply uterine fibroid. Diagnosis and treatment of fibroid by embolization of these branches of the uterine artery relies on fluoroscopy and injection of large amounts of contrast agents into the uterine vasculature. Typically the physician first examines the fibroid via angiography. Using a guide wire, a catheter is then inserted near the uterine artery. Once the catheter is in place, the guide wire is removed and contrast agent is injected into the uterine artery. The patient is then subjected to fluoroscopy or X-rays. In order to create an occlusion, an embolic agent, usually comprising tiny plastic particles, is introduced into the uterine artery via an angiographic catheter. The embolic agent is carried by the blood flow in the uterine artery to the vessels that supply the fibroid. In one method, polyvinyl particles are placed into the uterine artery at a point just before the nexis of vessels which spread into the uterine tissue. The mechanisms of occlusion are not well understood, some rely on thrombosis, others on mechanical occlusion. The particles flow into these vessels and clog them, thus disrupting the blood supply to the fibroid. In order for the physician to view and follow the occlusion process, contrast agent is injected subsequent to infusion of the embolic agent. The contrast agent is then detected by fluoroscopy. After reflux of the contrast agent, which signifies the column formation at the occlusion point and which is indicative of successful occlusion of the vessel, the physician stops further infusion of the embolic agent (Yamada et al., 1996, Cardiovascular and Interventional Radiology, p.139-145, Springer-Verlag, New York Inc.) Almost immediately following the procedure, the symptoms of heavy bleeding and pelvic pain are usually diminished, even though there may be post embolizaton pain caused by the procedure, which may last for 24-48 hours.

The current procedure for uterine fibroid embolization is problematic in many ways. First, since this procedure involves angiography which requires the use of fluoroscopy such as X-ray and injection of great amount of radiopaque contrast agents in the uterine vasculative, it is not desirable for use in certain patients such as pregnant women. Echography which does not involve X-ray, is preferable over angiography especially when treating the pelvic region in pregnant women or women of child-bearing age. Second, the physician relies on the reflux of contrast agent to monitor the embolization process. Since the embolic agent cannot, itself, be visualized, the monitoring of the embolization process is imprecise and is dependent upon the indirect viewing by means of large amount of often undesirable contrast agents. Third, because of the inability to properly visualize the embolic agent, some of the embolic and the contrast agents do not flow through the vessels supplying the fibroid and there is a consequent risk of collateral damage to other organs, which may result in serious medical complications. Therefore, more precision is required to assure delivery of the embolic particles only to the targeted uterine arteries. Imprecision of the embolic process necessarily increases the amount of embolic agent and contrast agent currently used for embolization. Additionally, the contrast agent is to a certain extent toxic, and is not recommended for women who wish to remain fertile. Other side-effects, such as allergic reactions to the contrast agent, have been documented. Finally, patients often experience very severe post-embolization pain which may last for 24 hours, which is caused by the contrast agent.

Similarly, when renal artery occlusion is performed for the treatment of renal cell cancer using gelatin sponge fragments with fluoroscopy and periodic angiography, characteristic complications of the procedure, consisting primarily of pain, fever, and gastrointestinal problems, the postinfarction syndrome, occur sometimes after radiography is completed and is believed due to the need to use a significant amount of contrast agents in combination with embolic agents and the inability under those circumstances to properly monitor the progress of the embolization procedure.

There is a lack of ideal embolotherapy agents. Therefore, there is a need for an embolic agent or composition for creating an embolus or occlusion in a controlled embolization process without the use of fluoroscopy or harmful contrast agents.

### 3. SUMMARY OF THE INVENTION

The present invention is aimed at providing an embolic particle and an embodiment kit comprising microbubbles that may be visualized by electromagnetic and/or sound waves. Such embolic particle, agent or composition is designed to be effective in embolization or occlusion of vessels or ducts in the body and at the same time be visible under ultrasound. This method would circumvent the undesirable side-effects of fluoroscopy (e.g., X-ray imaging) and the use of existing contrast agents in angiography. In particular, the synthetic embolic particle, agent or composition comprises microbubbles that are visible under ultrasound. This particle, agent or composition, thus, dramatically simplifies the process of embolization and makes available a particle, agent or composition that is useful in embolization and allows detection by electromagnetic and/or sound waves.

The present invention solves the above problem by an embolic particle according to claim 1 and an embolization kit according to claim 9. Preferred embodiment are described in the dependant claims.

There are many methods by which embolic particles, agents or composition of the present invention may comprise one or more microbubbles, detectable by ultrasound. By way of example only, first the microbubbles may be an integral structure of the embolic particles. Second, microbubbles may be present in the space between the embolic particles when these particles aggregate. Third, microbubbles may be adsorbed onto the surfaces of the particles. Fourth, microbubble forming contrast agent can be mixed with embolic particles in a composition.

Accordingly, the present invention provides embolic particles, for effecting embolization and occlusion, which particles comprise one or more voids rendering the embolic particles visible by ultrasound when the voids are filled with gas or microbubbles of gas. In specific embodiments, the voids comprise low pressure gas or a vacuum. In a specific embodiment, the pressure in the voids is between 10⁻⁶ atmosphere to 1 atmosphere. The embolic particles can be made of any material known in the art. In specific embodiments, the embolic particles are made of silicone, polyester, vinyl polymer, fibrin, cellulosic, alginate, polyvinyl alcohol (PVA), gelatin, acrylate polymer, collagen, polyolefins, polyethers, polystyrene, polyurethane, ethylene vinyl alcohol, polyethylene polymer (PVP), polylactic acid (PLA), polymethylmethacrylate (PMMA), polyethylene glycol (PEG) or any copolymer of these. In a preferred embodiment, the embolic particles are made of PVA. In a preferred aspect of the invention, the voids of the embolic particle contain one or more gases. In specific embodiments, the gas may be carbon dioxide, air, nitrogen, oxygen, nitrogen oxide, nitrous oxide, helium, argon, sulphur hexafluoride, methane, acetylene or carbon tetrafluoride.

The invention further provides embolic agents for effecting embolization and occlusion, which agents comprise embolic particles and gas or microbubbles of gas such that the embolic agent is visible under electromagnetic and/or sound waves such as ultrasound. The embolic particles may form the wall or membrane of microbubbles that traps gas. In a preferred embodiment, the embolic agent is formed by microencapsulation.

The invention further provides an embolic composition for effecting embolization and occlusion, which composition comprises embolic particles, one or more microbubbles generating contrast agent, and a compatible carrier. The contrast agent may be microbubble-generating microparticles of any kind known by those skilled in the art. See U.S. Patent No. 5,827,502, and U.S. Patent No. 5,795,562 for microbubble-generating contrast agent. In a preferred embodiment, the embolic composition comprises embolic particles, sugars, and surfactants.

The invention further provides methods of treatment using the embolic particle, agent or composition of the invention. For example, the embolic particle, agent or composition of the invention can be used in the treatment of internal bleeding, bulking, and solid tumor or cancer. In preferred embodiments, the methods of the present invention are used to treat solid tumor, liver cancer, urinary bulking, arteriovenous malformations, varicocele and pelvic congestion syndrome. In the most preferred embodiment, the embolic particle, agent or composition of the invention can be used in the treatment of uterine fibroids.

The invention further provides methods for detection or diagnosis using the embolic particle, agent or composition of the invention. In a preferred embodiment, the embolic particle, agent or composition of the invention is detected by magnetic resonance imaging (MRI). In the most preferred embodiment, the embolic particle, agent or composition of the invention is detected by ultrasound.

The invention also provides therapeutic and diagnostic kits and pharmaceutical compositions containing the embolic particle, agent, and composition of the present invention.

The invention further provides methods for detecting an embolic occlusion comprising introducing to the site to be occluded embolic particles and microbubbles; and visualizing the microbubble using ultrasound.

### 4. BRIEF DESCRIPTION OF THE FIGURES

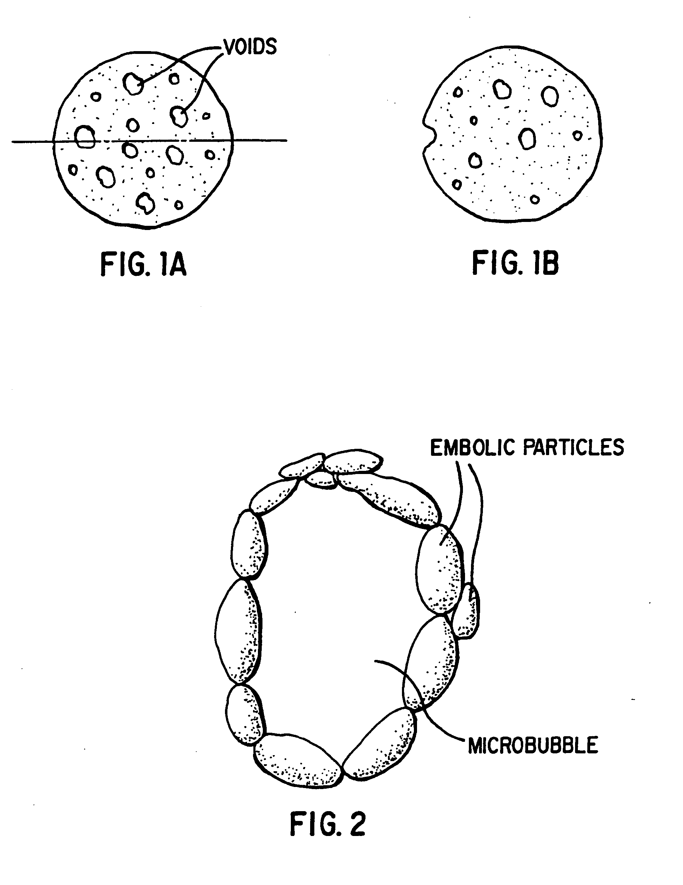

Figure 1A-B. (A) A schematic diagram showing the structure of an embolic particle. Each particle comprises voids at the surface. The voids at the surface contain gas or microbubbles of gas. (B) A schematic diagram of a cross-sectional view showing the interior of the embolic particle.
Figure 2. A schematic diagram showing the structure of an embolic agent where a microbubble is entrapped by the embolic particles.

### 5. DETAILED DESCRIPTION OF THE INVENTION

### 5.1. EMBOLIC PARTICLE

The present invention provides embolic particles for effecting embolization and occlusion, which particles comprise one or more voids in the particle for containing gas or microbubbles of gas such that the embolic particle is visible under ultrasound.

Any material known in the art as a suitable embolic particle or agent may be used to make the embolic particles according to the present invention. For general reviews of materials suitable for making the embolic particles see Goodwin, 1999, International symposium on endovascular therapy, Eleventh annual, p. 303-306. Depending on the type of material, the embolic particle can be rigid or elastic. When the particles are made of rigid material, they may be packed tightly to form an emboli. When the particles are made of elastic material, they may be compressed to a smaller diameter and/or packed tightly to form an emboli. In addition, when the particles are made of a material that is thromobogenic, occlusion occurs via thrombosis. These materials include, but are not limited to, plastics, polystyrene, polyurethanes, polyvinyl alcohol (PVA), gelatin, acrylate polymer or copolymer, ethylene vinyl alcohol, polyvinyl polymer (PVP), polylactic acid (PLA), polymethylmethacrylate (PMMA), polyethylene glycol (PEG), polymethylene oxide, polyethyleneoxide, polypropylene oxide as polyalkyleneoxide, medical grade silicone resins, particles of the polymeric acetate, vinylpyrrolidon, methylmethacrylate, fibrin foam, gelatin foam, polyurethane foams such as hypol (W.R. Grace & Co.), alkyl cyanoacrylate material, particles of acetylcellulose, acetylphtalylcellulose, polyvinyl and isopropyl palmitate (See Thelen et al., 1976, Fortschr. Rontgenstr. 124(3): 232-35), collagen, including but not limited to, microfibrillar collagen, Hemostatic sponge (Vitaphore Corp.), Vitacol^{™}, Semex collagen powder (Semex Medical, Inc.), polysiloxanes, metal powders, finely dispersed silica, finely dispersed polymers such as microcrystalline cellulose, polyethylene, polytetrafluoroethylene, cross-linked polymers such as cross-linked hyaluronic acid, agarose, and ion-exchange resins.

One of the preferable material for the embolic particle is polyvinyl alcohol. Polyvinyl alcohol may be either in powder form or sponge/foam form. It acts as a permanent or temporary embolic agent depending on whether non-resorbable or resorbable PVA is used. In the sense that the particles are not absorbed by the body, they are insoluble, and generally remain in place. The embolic particle of the invention may be in a sponge form produced commercially such as those sold by Merocel Corporation. In another embodiment, polyvinyl alcohol foam may be used to make the embolic particle of the invention. Polyvinyl alcohol foam particles are available as PVA from Cook, Inc., Bloomington, Indiana, as "Trufill" from Cordis, Miami, Florida, as "Contour" from Target Therapeutics, Fremont, California, and as "Ivalon" from Ivalon, Inc., San Diego, California. PVA foam can be obtained in a large range of sizes from approximately 25 microns to approximately 2,800 microns.

In another embodiment, Gelfoam® particles may be used to make the embolic particle of the invention. Gelfoam® particles are available as a powder or in sheets from Pharmacia and Upjohn in Kalamazoo, Michigan. The powder comprises particles which are 40-60 microns in size and are effective for distal devascularization of tumors. Gelfoam® particles are known as a temporary embolic agent because an occlusion generally lasts only weeks to months, which is advantageous in some situations such as in the treatment of gastrointestinal bleeding or in preoperative use where it is desired that the occlusion not be permanent.

The embolic particle may be of a wide variety of shapes, including but not limited to regularly or irregularly shaped, spherical, spheroidal, cylindrical or conical. In a preferred embodiment, the embolic particle is spherical. In a most preferred embodiment, the embolic particle is spheroidal.

The embolic particle can be of various sizes; for example, from 150-500, 500-700, 700-1500, 1500-2000, 2000-2500 µm. In a preferred embodiment, the embolic particle has a size range of 150-350, 350-500, 500-700, 700-1200, 1200-1500, and 1500-1700µm. In the most preferred embodiment, the embolic particle has a size range of approximately 355-500µm or 500-710µm.

The surface characteristics of the embolic particle may affect the thrombogenic properties and tendency to cause embolisms. In an embodiment of the invention, the embolic particle may have various surface characteristics, such as rough, irregular or smooth surfaces.

In a preferred embodiment, the embolic particle of the invention has one or more voids on its surface, the purpose of the voids being to contain gas or microbubbles of gas so as to render the embolic particle visible under ultrasound.

In a further embodiment of the invention, the embolic particle may have voids present throughout the particle. In preferred embodiments, the embolic particle of the invention has 1-5, 5-10, 10-20, 20-50, or 50-100 voids. The voids may be present within the particle as well as on the surface. These voids may be indentations, perforations, apertures, abyss, burrows, cavities, depressions, craters, fissures, furrows, gaps, niches, orifices, shafts, slits, slots, or space. In the more preferred embodiment, the embolic particle has one or more voids within the particle. The particular advantage of these particles is that they are stable gas-containing particles *in vivo.* In other embodiments, the voids comprise low pressure gas or a vacuum. In a specific embodiment, the pressure in the voids is between 10⁻⁶ atmosphere to 1 atmosphere.

The voids may be of any size ranging from 0.01-1, 1-5, 1-10, 10-15, 15-30, 30-50 microns. In preferred embodiments, the size of the voids ranges from 1-10 microns. In a more preferred embodiment, the size of the voids is less than about 10 microns, most preferably less than about 7 microns.

The total combined volume of the voids of the embolic particle may vary as a percentage of the total volume of the embolic particle. The volume of the voids may be 1-5, 5-10, 10-20, 20-50, 50-90% of the volume of the embolic particle.

The voids may be filled with various gases or microbubbles of gas, preferably gases which are known as suitable for diagnostic imaging. In other embodiments, the voids comprise low pressure gas or a vacuum. In a specific embodiment, the pressure in the voids is between 10⁻⁶ atmosphere to 1 atmosphere. These gases includes, but are not limited to, carbon dioxide, air, nitrogen, oxygen, nitrogen oxide, nitrous oxide, helium, argon, sulphur hexafluoride, methane, acetylene or carbon tetrafluoride. The term "gases" as used herein includes any substances at least partly in a gaseous form at 37°C.

One skilled in the art will readily understand how to make an embolic particle with one or more voids either on the surface or within the particle. Several methods are available for creating voids within the spheres, which voids are filled with various gases or microbubbles of gases. In one method, a leeching procedure is employed, in which salt or other aqueous soluble material is suspended in a polymer solution. After emulsification and solvent extraction/evaporation, the resultant spheres are incubated in aqueous medium overnight to leech out the soluble material. In another method, a double elmusion procedure is employed. A small amount of water is dispersed in a fine emulsion using means such as sonication within an organic polymer solution. The resultant emulsion is subsequently dispersed within an aqueous phase using a mixer of lesser power to form a water in oil in water emulsion and undergoes solvent extraction/evaporation. The resultant spheres are lyophilized overnight and dried in a vacuum oven to remove the water from the inner emulsion. In a third methodology, large spheres are produced using the above-mentioned emulsion and solvent evaporation/extraction procedure, but the emulsion droplets are made very large by reducing the rate of mechanical mixing during emulsification or by increasing the viscosity of the organic phase. The polymer precipitates at the interface between the aqueous and oil phases, and a capsule is formed with a hollow interior. The spheres are then lyophilized and dried in a vacuum oven to remove residual solvent.

In one embodiment, to manufacture the embolic particle of the present invention, a block of polymer manufactured by standard techniques usually entraps microbubbles of various sizes during the plastic working of the polymer material. When soft polymer is utilized, the soft polymer block is initially freeze dried. Then, any conventional micronisation techniques such as grinding, ball milling, or sonication for modifying and controlling particle size may be employed in making the embolic particle of the invention. The use of these methods results in a very broad range of particle diameters. The particles are then subjected to a sieving process where different size ranges of particles are obtained. Embolic particles having an average particle size of, for example, 40-3000 microns can be obtained.

In one embodiment, the gas is entrapped within an embolic particle and on the surface of the embolic particle when the particle is manufactured. Still further, the gas within a void is in the form of free gas microbubbles generated *in vivo* after the embolic agent is introduced into the patient. In other embodiments, the voids comprising low pressure or a vacuum are formed in a low pressure environment. In another embodiment, the gas is generated immediately before the embolic agent is introduced into the patient. Any method known in the art may be used to generate and/or stabilize gas microbubbles within the voids of the embolic particle. Any method known in the art may be used for diagnostic imaging such as ultrasound imaging (including regular ultrasound and doppler ultrasound-which detects blood flow velocity), magnetic resonance imaging or x-ray. (Schlief et al., 1990, Circulation Supplement III 82:28; Schartl et al., 1990, Circulation Supplement III 82:26I; Fritzsch et al., 1990, Inveset. Radiol 25(suppl): 160-161; Schlief et al., 1990, Echocardiography 7:61-64; Loughery et al., 1990, Echocardiography 7:279-292; Smith et al., 1989, JACC 13: 1622-1628).

In some embodiments, the voids on the surface of the embolic particle may in addition to gas or microbubbles of gas also be filled with various biologically active agents or drugs. These include growth factors, antimitotic agents, antioxidants, antimetabolite agents, antimicrobial agents, antibiotics, anti-cancer agents, anti-inflammatory agents and oligonucleotides. Antimitotic agents and anti-metabolite agents include methotrexate. Antimicrobial agents include, but are not limited to, antibacterial agents, antifungal agents or antiviral agents. The voids may also include antibiotics such as ampicillin, cefoxitin, oxacillin, amoxicillin, clarithromycin, doxycycline, erythromycin, ofloxacin, penicillin, vancomycin, and azithromycin.

### 5.2. EMBOLIC AGENT

The invention further provides embolic agents for effecting embolization and occlusion, which agents comprise embolic particles and gas or microbubbles of gas such that the embolic agent is visible under electromagnetic and/or sound waves such as ultrasound. In this aspect of the invention, the embolic particles form the wall or membrane that entrap a gas or microbubbles of gas so as to make the embolic agent visible under ultrasound.

The embolic agents of the invention may be prepared in a number of ways known in the art. Thus, for example, microencapsulation techniques for the preparation of microcapsules having a wall or membrane of polymeric material are described in literature such as "Microencapsulation and Related Drug Processes" by P.D. Deasy, Marcel Dekker Inc. New York (1984). For other useful methods of preparing microbubbles, see EP-A-0458745, US 5,795,562, US 5,827,502.

One method, the interfacial deposition technique, comprises dissolving or suspending the polymeric material used for the embolic agent in a water-immiscible low boiling organic solvent, emulsifying the resulting solution or suspension in an aqueous phase (preferably containing a surfactant to stabilize the resulting oil in water emulsion), and subsequently removing the organic phase (e.g. by evaporation or lyophilisation, preferably under an atmosphere of the gas which is desired to be incorporated) whereby the polymeric material forms a membrane at the interface between the aqueous and organic phases.

The size of the microbubbles so formed can be controlled by adjusting the stirring speed during emulsification and the nature of the surfactant which may be selected from Table III *infra*. Conventional additives may also be incorporated into the polymeric material; thus, for example, polyethylene glycols may be introduced to modify the flexibility and/or polarity of the membrane.

In an alternative method, the polymeric material that is used to make the embolic agent of the invention may be dissolved in an appropriate organic solvent (e.g. methylene bichloride, dimethyl sulphoxide, tetrahydrofuran or dimethylformamide) and then dispersed (e.g. using a high speed stirrer) in an aqueous phase (preferably containing a polymeric material such as polyvinyl alcohol or a poloxamer) so as to precipitate particulate polymeric material which may be collected and lyophilized to yield an embolic agent in accordance with the invention. See EP-A-0458079. Variants for the preparation of the embolic agent include injecting the organic polymer solution, preferably together with a physiologically acceptable stabilizer such as hydroxypropyl cellulose, into liquid nitrogen. Alternatively the material used for the embolic agent may be dissolved in an appropriate organic solvent (e.g. methylene chloride or tetrahydrofuran), followed by spray drying of the solution, or of an oil in water or water in oil emulsion of the organic polymer solution with an aqueous phase. The embolic agent in accordance with the invention may also be prepared using coacervation of double emulsion techniques. See WO 91/12823.

In one embodiment, the embolic agent comprises aggregates of embolic particles that entrap gas or microbubbles of gas. Any conventional micronisation techniques such as grinding, ball milling, or sonication for modifying and controlling particle size may be employed in a method of making the embolic agent of the invention. Ball-milling of the solid mixture has been found to be particularly advantageous, permitting the preparation of embolic particles to aggregate (for example having an aggregate size of 200-1250 microns, such as 300-500 microns). Each embolic particle having a particle size of, for example, 10-500 microns, such as 10-100 microns. Such aggregates will tend to contain a substantial volume of gas adsorbed on their surfaces and entrained in voids such as interparticle cavities or at grain boundaries between the crystallites. The embolic particles may be coated with, for example, polyethylene glycol units, proteins or polysaccharide to modify their aggregation tendencies and/or biological properties. The particle size may, for example, be selected to be substantially commensurate with the desired microbubble size. In ultrasonic applications, to achieve resonance with the preferred imaging frequencies of about 0.1-15 _{MHZ}, it may be convenient to employ microbubbles having an average size of 0.1-10 µm, *e.g.* 1-7 µm. Substantially larger bubbles e.g. with average sizes up to 500 µm, may however be useful in other applications, for example gastrointestinal imaging.

In one embodiment, the embolic agent comprises adhesive glues and polymerizing agents. These include N-butly-cyanoacrylate (NBCA) and IBCA, ethylene vinyl alcohol mixed with metrizamide or tantalum (Embolyx^{™}) in dimethyl sulfoxide, and hydrogel casting agents. The method of making the embolic agent comprises adhesive glues and polymerizing agents consists sonicating the adhesive glues and polymerizing agents to introduce gas bubbles into the embolic solution prior to injection to make it visible under ultrasound. Bubbles may be stabilized via a viscous agent or a surfactant. The radiopaque elements present in this liquid embolic agent may be removed prior to injection.

### 5.3. EMBOLIC COMPOSITIONS AND FORMULATIONS

The invention further provides an embolic composition for effecting embolization and occlusion, which composition comprises embolic particles, and a gas or microbubbles of gas such that the composition is made visible under ultrasound; and a compatible carrier. Alternatively, the invention also comprises an embolic composition for effecting embolization and occlusion, which comprises embolic particles; ultrasound contrast agent that generate microbubbles such that the composition is made visible under ultrasound; and a compatible carrier.

The microbubble generating ultrasound contrast agent used in the present composition may be any ultrasound contrast agent that are available commercially. These include but not limited to, Albunex® from Molecular Biosystems, Inc., Echovist® from Schering-Plough Pharmaceuticals, and EchonGen® from Sonus Pharmaceuticals.

The microbubbles may be generated by microbubble-generating microparticles known by those skilled in the art. Carroll et al., 1980, Investigative Radiology 15:260-266. See U.S. Patent No. 5,795,562 and Example 6.5 *infra.*

Techniques involving the use of sugars in ultrasound contrast agents are described in, for example, U.S. Pat. No. 5,827,502, U.S. Pat. No. 4,681,119, U.S. Pat. No. 4,442,843 and U.S. Pat. No. 4,657,756. EP-A-0123235 and EP-A-0122624 suggest ultrasound contrast agents consisting of surfactant-coated or surfactant-containing gas-containing microparticles which may include a variety of sugars. Where surfactant-containing microparticles are described, these are prepared simply by commingling the surfactant with the microparticulate materials, *e.g.*, by trituration. See Section 6.5 *infra,* Table III, and IV.

Accordingly, one embodiment of the embolic composition of the invention comprises embolic particles; sugars and surfactant for forming gas or microbubbles of gas so as to make the composition visible under ultrasound; and a compatible carrier.

The sugars that are suitable to form the microbubbles are well known in the art including, but not limited to, hexoses such as glucose, fructose or galactose; disaccharides such as sucrose, lactose or maltose; pentoses such as arabinose, xylose or ribose; and polysaccharide such as α , β and γ cyclodextrins, maltodextrin and glycogen. The term "sugar" as used herein is also intended to include sugar alcohols, *e.g.* alditols such as mannitol or sorbitol. Microparticles of the above sugars will normally have gas present as an inclusion in the voids of their crystal structure and/or adhered to their surface, which gas may generate microbubbles when, for example, the microparticles are suspended or dissolved in an injectable carrier liquid, for example water for injection, an aqueous solution of one or more inorganic salts (*e.g.* physiological saline or a physiological buffer solution), an aqueous solution of a monosaccharide (*e.g.* glucose or galactose) or disaccharide (*e.g.* lactose), or an aqueous solution of a physiologically tolerable monohydric or polyhydric alcohol (*e.g.* ethanol, propanol, isopropanol, ethylene glycol, propylene glycol, glycerine or polyethylene glycol).

The term "surfactant" as used herein means any compound having amphiphilic properties capable of modifying surface tension. The amount of surfactant used may be determined by one skilled in the art. In one embodiment, the embolic composition of the present invention comprises 0.01%-2% weight ratio of surfactant to embolic composition or the surfactant may, for example, be present in an amount of 0.01-5.0 wt. %, preferably 0.1-2.0 wt. %, relative to the embolic particles.

A wide variety of surfactants may be used in the embolic composition of the invention; the surfactant chosen is required to be biocompatible, i.e., it should be physiologically tolerable in the quantities in which it is to be administered.

The surfactant may, for example, be an amphiphilic lipid, *e.g.*, selected from fatty acids and salts (*e.g.*, alkali metal salts) thereof, steroid acids, sterols, phospholipids and glycolipids. Such lipids include high molecular weight (*e.g.*, C₁₀₋₅₀) straight chain saturated and unsaturated aliphatic acids, such as capric, palmitic, hexadecanedioic, stearic, linolenic, behenic, docosanedioic and melissic acids; aralkanoic acids, *e.g.*, phenyl lower alkanoic acids such as 2-phenylbutyric acid; salts of any of the foregoing acids; mono- and diglycerides, for example glyceryl esters of high molecular weight (*e.g.*, C₁₀₋₅₀) aliphatic acids, such as glyceral monolaurate; cholanic acids such as 5β-cholanic acid, cholesterol; sorbitan esters of fatty acids such as Span-type materials; high molecular weight (*e.g.*, C₁₀₋₅₀) straight chain aliphatic alcohols such as stearyl alcohol and acetyl alcohol; phospholipids such as phosphatidyl choline (lecithin) and dioleoylphosphatidyl ethanolamine (DOPE); and mixtures thereof. See also DE-A-3834705.

Other surfactant which may be employed include anionic surfactant, for example alkali metal sulphate such as sodium lauryl sulphate and sulphonated esters such as sodium dioctyl sulphosuccinate (docusate) and non-ionic surfactants, for example polyoxyethylene-polyoxyproplyene copolymers (*e.g.* poloxamers such as Pluronic F68) and polyoxyethylated sorbitan esters (*e.g.* polysorbates such as Tween-type material).

In a specific embodiment, the embolic composition of the present invention comprises microparticulate microbubble-generating contrast agent. The weight ratio of the contrast agents to the embolic particles and other components in the composition may be determined by one skilled in the art. The weight ratio may be adjusted according to the degree of ultrasonic image resolution desired. It should be noted that if the amount of contrast agent in the preparation is below 0.005% by weight, the particles become difficult to monitor during the process of embolization. On the other hand, if the amount of contrast agent rises over 8% by weight, the composition becomes less mobile. Its apparent viscosity rises and the physical and mechanical properties of the particles may deteriorate. This composition may become more dense and difficult to handle during infusion.

In a preferred composition, the embolic particles and a microbubble-generating contrast agent are each suspended in appropriate mutually miscible solvent.

In a specific embodiment, the embolic particle and the microparticulate microbubble-generating contrast agent may be coupled. They may be coupled by any bonds that are known in the relevant art including but not limited to, Van de Waals force, ionic bond, covalent bond, hydrogen bond or chemical cross-linking.

The embolic particles are immersed in a sterile physiological solution. A suitable sterile physiological solution can contain various agents which can be determined by one skilled in the art. The carrier that is suitable for use in the invention has to optimize the suspension properties of the particulate agent and preserve the function of a microbubble-generating agent. These include, but not limited to saline, stabilizing (viscous) agent or surfactant as described above.

The weight ratio of the embolic particles to physiological solution may be determined by one skilled in the art. The weight ratio of the ingredients of the proposed composition for embolization of blood vessels is limited by the fact that when the amount of embolizing particles in the composition is less than 0.01%, too much liquid is injected into the artery, possibly allowing emboli to stray into lateral vessels. This may result in undesired embolization of vital organs and tissues. When the embolizing particles comprise greater than 15% of the composition, the catheter or the needle can be easily clogged. Accordingly, in an embodiment of the composition of the present invention, the weight ratio of the embolic particle to the physiological solution is about 0.01-13% by weight. In a more preferred embodiment, the weight ratio of the embolic particle to the physiological solution is about 0.01-0.2%, or 0.2-2% by weight.

In a preferred embodiment, the density of the physiological solution is close to that of the particles for even suspension. Typically, 100mg of particles is mixed with 10cc to 30 cc of liquid mixture. In a specific embodiment, PVA embolic particle of this invention may be administered with 100 mg of PVA and 20 cc of normal saline. Dextran and/or albumen may also be added to get a better suspension of PVA. The distance traveled by the embolic particles depends on the concentration of the embolic mixture. Dilute mixtures travel more distally from the point of administration whereas concentrated mixtures occlude more proximally.

In another embodiment, the embolic composition of the present invention comprises in weight percent, 0.01-2% of polyethyleneoxide. The presence of this compound is known to improve the dispersion of embolic particles and prevent clogging of syringe or catheter during injection. See U.S. Patent No. 4,999,188.

The embolic agent of the present invention may be administered as separate compositions or as a single composition with more than one kind of embolic particles that are described supra. Additionally, the therapeutic value of the composition of the invention may be augmented by its use in combination with drugs or toxins such as ricin or with chemotherapeutic agents such as methotrexate. In a preferred embodiment, the composition, if desired, can also contain minor amounts of wetting or emulsifying agent, or pH buffering agents.

The embolic agents of the invention may be stored and transported in dry form, in which condition they will normally be stable indefinitely, being mixed with an appropriate liquid carrier (e.g. sterile water for injection, physiological saline or phosphate buffer) prior to administration. In this way the concentration of the injected or otherwise administered embolic agent may be varied at will, depending on the precise nature of the application. They may also be stored in suspension.

Generally, the ingredients for the composition of the present invention are supplied either separately or mixed together in unit dosage form, for example, as a dry lyophilized powder or water free concentrate in a hermetically sealed container such as an ampoule or sachette indicating the quantity of active agent. An ampoule of sterile diluent can be provided so that the ingredients may be mixed prior to administration.

The invention also provides a pharmaceutical pack or kit comprising one or more containers filled with one or more of the ingredients of the composition of the invention. Associated with such container(s) can be a notice in the form prescribed by a governmental agency regulating the manufacture, use or sale of pharmaceuticals or biological products, which notice reflects approval by the agency of manufacture, use or sale for human administration.

The precise dose of the embolic composition of the present invention to be employed will depend on the size of the vessel that needs to be occluded, the nature of the disorder, and should be decided according to the judgment of the practitioner and each patient's circumstances according to standard clinical techniques. An effective amount is that amount sufficient to produce an occlusion or emboli at the appropriate site of the vessel or duct in the circulatory system or a duct system respectively. Effective doses may also be extrapolated from dose-response curves derived from animal model test systems.

### 5.4. THERAPEUTIC USES AND TARGET TREATMENTS

Methods for treatment of fibroids are well known to those skilled in the art (see, *e.g.*, Ravina et al., 1995, Fertilite, Sexualite 23:45-49; Ravina et al., 1995, Lancet 346:671-672; Ravina et al., 1997, Bull Acad Natl Med 181:233-243; Bradley et al., 1998, Brit J of Obst and Gynaec 105:235-240; Worthington-Kirsch et al., 1998, Radiology 208:625-629; Kuhn et al., 1999, Aust NZ J Obstet Gynaecol 39: 120-121; Abbara et al., 1999, JVIR 10:409-411; Berkowitz et al., 1999, Journal of Reproductive Medicine 44:373-376; Goodwin et al., 1997, Journal of Vascular and Interventional Radiology 8:517-526; Robert et al., 1997, Radiology 208:625-629; Phillips et al., 1997, J Am Assoc Gynecol Laparosc 4:425-433; Cirkel et al., 1992, Clin Ther 14(suppl A):37-50; Greenwood et al., 1987, Radiology 164:155-159; Gilbert et al., 1992, Am J Obstet Gynecol 166:493-497; Yamashita et al., 1994, Br J Radiol 67:530-534; Poppe et al., 1987, Am J Obstet Gynecol 156:179-180; Stancato-Pasik et al., 1996, Radiology 201(P)179; McIvor et al., 1996, Br J Radiol 69:624-629).

A preferred embodiment of the method of the present invention is the treatment of fibroids. Fibroids can grow within the uterine wall ("intramural" type), on the outside of the uterus ("subserosal" type), inside the uterine cavity ("submucosal" type), between the layers of broad ligament supporting the uterus ("interligamentous" type), attached to another organ ("parasitic" type), or on a mushroom-like stalk ("pedunculated" type). These fibroids range in size from a few millimeters to 40 centimeters. Accordingly, the method of the present invention can be used to treat subserosal fibroids, intramural fibroids, submucous fibroids, pedunculated fibroids, interligamentous fibroids or parasitic fibroids. The method of the present invention can be used to treat fibroids of various sizes which include 1-5mm, 5-15mem, 15-30mm, 30mm-100mm, and 10-40 centimers.

Another treatment of fibroids that has been shown to be effective is hormonal therapy. Accordingly, the method of the present invention encompasses the use of embolization composition in combination with agents employed in hormonal therapy. These include birth control pills, which can regulate the natural hormone balance and reduce bleeding symptoms; progesterone, which reduces the effects of the menstrual cycle on fibroids; and Lupron, which creates a "false" menopause by shrinking fibroid, as occurs during normal menopause.

In the treatment of fibroids, embolization of the entire uterine arterial distribution network is preferred. This is because it is impractical to selectively catheterize individual vessels supplying only fibroids, the major reason is that there are far too many branches for catheterization and embolization to be performed in an efficient and timely manner. Also, it is difficult to tell whether any one vessel supplies fibroids rather than normal myometrium. In many women, the fibroid of the uterus is diffuse, and embolization of the entire uterine arterial distribution affords a global treatment for every fibroid in the uterus.

Various other disorders may be treated by the method of the present invention. Such disorders include, but are not limited to, gastrointestinal bleeding, renal bleeding, pelvic fracture, pelvic congestion syndrome, urinary bleeding, varicose bleeding and intracerebral arteriovenous fistulas. Other examples of situations requiring occlusion include, but are not limited to, the occlusion of saphenous vein side branches in a saphenous bypass graft procedure, neurovascular occlusion, chemoembolization, aortic aneurysm corrective procedures, and chronic venous insufficiency treatment.

When an aneurysm is difficult to approach or has a higher surgical risk, the method of the present invention can be used as an alternative to surgery to treat such aneurysms. In a preferred embodiment, the method of the present invention can be used to treat subarachnoid hemorrhage.

Pulmonary artery pseudoaneurysms can occur as a result of erosion of the artery by an adjacent tuberculous cavity (Rasmussen aneurysm), blunt or penetrating thoracic trauma, neoplasms, improper placement of flow-directed pulmonary arterial (Swan-Ganz) catheters, and septic emboli. Causes of pulmonary artery aneurysms include Behcet's disease and Marfans syndrome. Accordingly, the method of the present invention can be used to treat pulmonary artery pseudoaneurysm.

Arteriovenous malformations (AVMs) are abnormal collections of blood vessels in the brain which shunt blood from a high pressure artery to a low pressure vein, resulting in hypoxia and malnutrition of those brain regions from which the blood is diverted. Accordingly, in one embodiment, the method of the present invention can be used to treat AVMs. In one embodiment, the method of the present invention can be used to treat cerebral arteriovenous malformations. More preferably, the embolization method of the present invention is used to effectuate an occlusion through a nidus.

Some patients suffer severe bleeding from the nose (epistaxis) which can become life threatening. Accordingly, one embodiment of the method of the present invention is to treat nose-bleed.

One preferred embodiment of the method of the present invention is the treatment of cavernous sinus dural arteriovenous fistula. In one embodiment, the method of the invention involves transarterial external carotid embolization. In another embodiment, the method of the invention involve transvenous embolization.

Another preferred embodiment of the method of the present invention is the treatment of arterioportal fistula.

Another preferred embodiment of the method of the present invention is the treatment of massive upper gastrointestinal (UGI) bleeding.

Another preferred embodiment of the method of the present invention is the treatment of uterine hemorrhage.

One embodiment of the method of the present invention is to treat varicocele. The method of the present invention is minimally invasive with a very short postoperative recovery and minimal pain. The effectiveness of the procedure can be measured by ultrasound, venography, scintigraph, infrared thermometry, and in some cases, sperm count, sperm motility and sperm morphology.

### 5.5. CANCER AND SOLID TUMOR TREATMENTS

Embolization using the composition of the invention can be used to treat various types of solid tumors in the body, a list of solid tumors are provided in Table 1.

**TABLE 1**

| | | |
|---|---|---|
| Solid tumors | | |
| | sarcomas and carcinomas | |
| | | fibrosarcoma |
| | | myxosarcoma |
| | | liposarcoma |
| | | chondrosarcoma |
| | | osteogenic sarcoma |
| | | chordoma |
| | | angiosarcoma |
| | | endotheliosarcoma |
| | | lymphangiosarcoma |
| | | lymphangioendotheliosarcoma |
| | | synovioma |
| | | mesothelioma |
| | | Ewing's tumor |
| | | leiomyosarcoma |
| | | rhabdomyosarcoma |
| | | colon carcinoma |
| | | pancreatic cancer |
| | | breast cancer |
| | | ovarian cancer |
| | | prostate cancer |
| | | squamous cell carcinoma |
| | | basal cell carcinoma |
| | | adenocarcinoma |
| | | sweat gland carcinoma |
| | | sebaceous gland carcinoma |
| | | papillary carcinoma |
| | | papillary adenocarcinomas |
| | | cystadenocarcinoma |
| | | medullary carcinoma |
| | | bronchogenic carcinoma |
| | | renal cell carcinoma |
| | | hepatoma |
| | | bile duct carcinoma |
| | | choriocarcinoma |
| | | seminoma |
| | | embryonal carcinoma |
| | | Wilms' tumor |
| | | cervical cancer |
| | | uterine cancer |
| | | testicular tumor |
| | | lung carcinoma |
| | | small cell lung carcinoma |
| | | bladder carcinoma |
| | | epithelial carcinoma |
| | | glioma, glioblastoma |
| | | astrocytoma |
| | | medulloblastoma |
| | | craniopharyngioma |
| | | ependymoma |
| | | pinealoma |
| | | hemangioblastoma |
| | | acoustic neuroma |
| | | oligodendroglioma |
| | | menangioma |
| | | neuroblastoma |
| | | retinoblastoma |

Accordingly, one embodiment of the method of the present invention encompasses the treatment of various types of solid tumors including, but not limited to, sarcomas, carcinomas, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, thyroid carcinomas, medullary carcinoma, bronchogenic carcinoma, renal cell carcinoma, hepatoma, bile duct carcinoma, choriocarcinoma, seminoma, embryonal carcinoma, Wilms' tumor, cervical cancer, uterine cancer, testicular tumor, lung carcinoma, small cell lung carcinoma, bladder carcinoma, epithelial carcinoma glioma, glioblastoma, astrocytoma, medulloblastoma, craniopharyngioma, ependymoma, pinealoma, hemangioblastoma, acoustic neuroma, oligodendroglioma, menangioma, neuroblastoma, and retinoblastoma.

The most preferred embodiment of the method of the present invention is the treatment of solid tumor, liver cancer, arteriovenous malformations, varicocele, pelvic congestion syndrome and various bulk-related symptoms such as urinary bulking.

Renal cell cancer, also known as kidney cancer or renal adenocarcinoma, is a disease in which cancer cells are found in the tubules of the kidney. Accordingly, a preferred embodiment of the method of the present invention is the treatment by embolization of renal cell cancer.

Other treatments of renal cell cancer that have been shown to be effective include surgery, chemotherapy, radiation therapy, hormonal therapy and biological therapy (biological response modifier therapy or immunotherapy). Accordingly, the method of the present invention encompasses the use of the embolization composition of this invention in combination with other treatment methods such as surgery, chemotherapy, radiation therapy, hormonal therapy or biological therapy.

It has been shown that embolization before a major surgery can reduce the amount of bleeding during a surgical procedure. In particular, preoperative embolization in highly vascular tumors can decrease intra-operative blood loss. Accordingly, one embodiment of the method of the present invention encompasses preoperative embolization. In the most preferred embodiment, gelform embolization is performed within 24 hours of surgery.

In other embodiments of the invention, the patient being treated for solid tumor or cancer with the method of the invention, optionally, may be treated with other cancer treatments such as surgery, radiation therapy or chemotherapy. In particular, the method of the invention used to treat or prevent cancer may be administered in conjunction with one or a combination of chemotherapeutic agents including, but not limited to, methotrexate, taxol, mercaptopurine, thioguanine, hydroxyurea, cytarabine, cyclophosphamide, ifosfamide, nitrosoureas, cisplatin, carboplatin, mitomycin, dacarbazine, procarbizine, etoposides, campathecins, bleomycin, doxorubicin, idarubicin, daunorubicin, dactinomycin, plicamycin, mitoxantrone, asparaginase, vinblastine, vincristine, vinorelbine, paclitaxel, and docetaxel. In a preferred embodiment, the embolic agent of the present invention is conjugated to a chemotherapeutic agent or other type of toxin (*e.g.*, a ricin toxin, or a radionuclide, or any other agent able to kill cancerous cells or to arrest cell growth).

Treatment of patients with hepatocellular carcinoma (HCC) is largely influenced by the clinical stage of the disease. While hepatic resection and liver transplantation offer the best chances of cure in patients with a single, small tumor, arterial embolization is the only recommendable palliative treatment of patients with large tumors and poor hepatic function. Accordingly, a preferred embodiment of the method of the present invention is the treatment of hepatocellular carcinoma.

In a preferred embodiment, the method of the invention is used to treat liver cancer. The embolic particle of the present invention is bound to chemotherapeutic agents that are bound to microspheres or contained within microcapsules (*e.g.* liposomes) or iodinized oils as the carrier.

Another embodiment of the method of the present invention is the treatment of diffuse toxic goiter. The method of the invention involves embolization of the thyroid arteries. In a preferred embodiment, the embolic particle of the invention is made of polyurethane. The efficacy of the treatment is shown when the thyroid gland decreases in size after treatment.

### 5.6. ASSESSMENT OF EFFICACY OF TREATMENTS

Whether a particular treatment of the invention is effective to treat a certain type of cancer can be determined by any method known in the art, for example but not limited to, those methods described in this section.

The safety and efficiency of the proposed composition for embolization of blood vessels may be tested in the course of systematic medical and biological assays on animals, toxicological analyses for acute and systemic toxicity, histological studies and functional examinations, and clinical evaluation of patients having a variety of indications for embolization.

The efficacy of the method of the present invention may be tested in appropriate animal models, and in human clinical trials, by any method known in the art. For example, the animal or human subject may be evaluated for any indicator of disease that the method of the present invention is intended to treat. Then, the efficacy of the method of the present invention for tumor treatment can be assessed by measuring the size of a tumor in the animal model or human subject at suitable time intervals before, during, or after embolization. Any change or absence of change in the size of the tumor can be identified and correlated with the effect of the treatment on the subject. The size of the tumor can be determined by any method known in the art (*e.g.*, by ultrasound or angiography). In another example, the efficacy of the method of the present invention for internal bleeding, such as ulcer, can be assessed by any method known in the art, such as measuring the amount of blood in the stool in the animal model or human subject at suitable time intervals before, during, or after embolization. Any change or absence of change in the amount of blood in the stool can be identified and correlated with the effect of the treatment on the subject. Other types of internal bleeding can be determined by any method known in the art (*e.g.*, angiography). In another example, the efficacy of the method of the present invention for aneurysm can be assessed by any method known in the art, such as a reduction of symptoms related to aneurysm before, during, or after embolization. Any change or absence of severe headache, nausea, vomiting, double vision or unconsciousness may be correlated with the effect of the treatment on the subject. Other improvements can be determined by any method known in the art.

In fibroid embolization, the patient may undergo transabdominal and endovaginal pelvic ultrasound examination prior to embolization as well as after embolization. Longitudinal, anteroposterior, and transverse measurements of the uterus and dominant fibroid may be obtained. Approximate uterine volume and volume of dominant fibroid can be calculated with use of the formula for a prolate ellipsoid, as previously described (Onisini et al, 1984, Radiology 153:113-116). Percent volume reduction can be calculated for each patient, and statistical comparison of pre-embolization and post-embolization uterine volumes can be accomplished (paired *t* test). Other symptoms may likewise be monitored, including but not limited to, abnormal bleeding, pelvic pain, abdominal or pelvic fullness, pressure in the colon and urinary bladder, causing constipation or frequent urination, and leg swelling etc. Likewise, the progress of a cancer or tumor before and after embolization may be monitored by assaying the levels of the particular cancer or tumor antigen (or another antigen associated with the particular cancer or tumor) either in the serum of the subject. Additionally, other imaging techniques, such as computer tomographic (CT) scan, sonograms, or any other imaging method, may be used to monitor the progression of the cancer or tumor both before and after embolization. Biopsies may also be performed. Before carrying out such trials in humans, the tests for efficacy of the method of the invention can be performed in animal models of the particular cancer or tumor.

### 5.7. EFFECTIVE DOSE

The compositions and embolic agents described herein can be administered to a patient at therapeutically effective doses to treat certain diseases. A therapeutically effective dose refers to that amount of the composition of the invention sufficient to result in an improvement in the treated subject.

Toxicity and therapeutic efficacy of composition of the invention can be determined by standard pharmaceutical procedures in experimental animals, *e.g.*, for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compositions which exhibit large therapeutic indices are preferred. While compositions that exhibit toxic side effects can be used, care should be taken to design a delivery system that targets such composition to the vessel or duct in order to minimize potential damage to normal cells and, thereby, reduce side effects.

The data obtained from animal studies can be used in formulating a range of dosage for use in humans. The dosage of such compositions lies preferably within a range of concentrations that include the ED₅₀ with little or no toxicity. The dosage can vary within this range depending upon the dosage form employed. Such information can be used to more accurately determine useful doses in humans.

The optimal dosage to be administered will be readily determined by those skilled in the art and will vary on the condition being treated, the particular type of embolic agent used and mode of administration. Other factors include the weight and condition of the human or animal. It is to be understood that the present invention has application for both human and veterinary use.

### 5.8. METHODS OF ADMINISTRATION

Different techniques of embolization may be employed for the introduction of the embolic particle, agent or composition of the present invention. These techniques include but not limited to, the use of a balloon with a calibrated leak (Kerber, 1976, Radiology 120:547-550), making embolic injections possible within reasonably safe distances with flow control; the use of microcatheter, such as Tracker which can be used for injection of particles such as PVA over a guidewire; the use of steerable catheter; and the use of a purely flow-guided microcatheter, such as Magic, for rapid injection with or without flow control.

Accordingly, the method of the present invention encompasses various ways of administering the embolic agent of the invention to effect an embolization. One skilled in the art can determine the most desirable way of administering the embolic agent depending on the type of treatment and condition of the patient. Many methods may be used to introduce the embolic agent; these include but are not limited to intravenous, percutaneous or any other standard routes of administration. The preferred embodiment of the method of the present invention is that the embolic agent may be delivered percutaneously (through a syringe) or through a catheter (Seldinger technique). The depth of penetration is dependent on the viscosity of the agent, the flow velocity of the vessel being embolized and the order of the vessel in which the delivery catheter is placed.

The embolization method of the present invention can also be used in various liquid-filled regions of the body, such as the lymphatic, digestive, urinary, reproductive, biliary and other systems, as well as inter-peritoneal, intra-cranial, intra-thoracic and other body cavities and spaces.

Embolization using the composition of the invention can be effected in various blood vessels in the body. See Table II which lists the major systemic arteries. Accordingly, one embodiment of the method of the present invention encompasses embolization of various blood vessels which include, but are not limited to axillary, brachial, brachiocephalic, celiac, common carotid, common iliac, coronary, deep femoral, digital, dorsalis pedis, external carotid, external iliac, femoral, gastric, hepatic, inferior mesenteric, internal carotid, internal iliac, left gastric, middle sacral, ovarian, palmar arch, peroneal, popliteal, posterior tibial, pulmonary, radial, renal, splenic, subclavian, superior mesenteric, testicular, and ulnar.

**TABLE II**

| Major Systemic Arteries | |
|---|---|
| Artery | Body Areas Supplied |
| Axillary | Shoulder and axilla |
| Brachial | Upper arm |
| Brachiocephalic | Head, neck, and arm |
| Celiac | Divides into left gastric, splenic, and hepatic arteries |
| Common carotid | Neck |
| Common iliac | Divides into external and internal iliac arteries |
| Coronary | Heart |
| Deep femoral | Thigh |
| Digital | Fingers |
| Dorsalis pedis | Foot |
| External carotid | Neck and external head regions |
| External iliac | Femoral artery |
| Femoral | Thigh |
| Gastric | Stomach |
| Hepatic | Liver, gallbladder, pancreas, and duodenum |
| Inferior mesenteric | Descending colon, rectum, and pelvic wall |
| Internal carotid | Neck and internal head regions |
| Internal iliac | Rectum, urinary bladder, external genitalia, buttocks muscles, uterus and vagina |
| Left gastric | Esophagus and stomach |
| Middle sacral | Sacrum |
| Ovarian | Ovaries |
| Palmar arch | Hand |
| Peroneal | Calf |
| Popliteal | Knee |
| Posterior tibial | Calf |
| Pulmonary | Lungs |
| Radial | Forearm |
| Renal | Kidney |
| Splenic | Stomach, pancreas, and spleen |
| Subclavian | Shoulder |
| Superior mesenteric | Pancreas, small intestine, ascending and transverse colon |
| Testicular | Testes |
| Ulnar | Forearm |

### 5.9. DIAGNOSTIC METHODS

The present invention allows therapeutic and diagnostic goals to be accomplished in one process. The present invention has the advantage of embolizing the vessel or duct of interest and visualizing the embolic agent so as to follow the process of embolization and determine the efficacy of the process simultaneously.

The compositions of the present invention allow visualization of the embolization procedure, enabling the surgeon to monitor the procedure while it is being performed.

In accordance with a specific embodiment of the method of the present invention, the composition of the present invention is introduced into the blood vessel, generally by injection, catheterization or the like as described in Section 5.8 supra. The embolic agent is then detected by ultrasound where its position, as well as the blood flow rate, can be determined by measuring the positions and velocities of the gas or microbubbles of gas. Successful embolization is indicated by the fact that the microbubbles, as detected by ultrasound, are stationary after arriving at the desired site. In general, one of skill in the art would determine the method of visualization and the particular type of machine to be used depending on the type of embolic particle, agent or composition used in the present invention. For a review of visualization of microbubbles through ultrasound, see Ophir et al., 1980, Ultrasonic Imaging 2:67-77; Parker et al., 1987, Ultrasound in Med. & Biol. 13(9):555-566; Gramiak et al., 1968, Invest. Radiol. 3:256; Gramiak et al., 1969, Radiology 92:939; Ziskin et al., 1972, Invest. Radiol. 6:500-505; Marich et al., 1975, J. Clin. Ultrasound 3:5-16; Goldberg, 1976, Radiol. 118:401-404; Carroll et al., 1979, Invest. Radiol. 5:374; Shung et al., 1976, IEEE Trans. Biomed. Engin. BME 23:465; Sigelmann et al., 1973, J. Acoust. Soc. Amer. 53:1351-1355; Carstensen et al., 1953, J. Acoust. Soc. Amer. 25:286-289.

One preferred embodiment of the method of the present invention utilizes state-of-the-art diagnostic ultrasound equipment, which gives high sensitivities and specificities that are above 70%, to detect the embolic agent of the present invention. Other methods of detecting the embolic agent of the present invention may be employed by one skilled in the art. Methods which may be used to detect the transmission characteristics of electromagnetic and sound (elastic wave) radiation transmitted through a body fluid include medical ultrasonic imaging (both transmission and echographic), X-ray imaging (e.g. CT scanning), nuclear magnetic resonance (NMR) imaging, magnetic resonance image (MRI) and microwave imaging.

### 6. EXAMPLES

### 6.1. Microbubble-Generating Composition

The following examples set forth exemplary protocols for preparing microbubble-generating composition using the method as described in U.S. Patent No. 5,827,502. However, one of ordinary skill in the relevant art would recognize that the technique of these examples can be utilized to prepare other microbubble-generating compositions.

D-(+)-galactose (10 g) is dissolved in distilled water (14 g) at 50°C. The solution is then sterile filtered and cooled on ice. The stated amounts of the surfactant (in % w/w relative to the galactose) listed in Table III are each dissolved in the amount of 96% ethanol (or water in Examples 5 and 6) shown in Table III, heated to 50-78°C, and the resulting solution is sterile filtered and then aseptically added to the cold aqueous galactose solution under stirring. The resulting mixture is evaporated to dryness at 40°C under reduced pressure (10 torr), and the resulting solid product is dried in a desiccator overnight and then ground for 10 minutes under aseptic conditions in a stainless steel ball mill having a 50ml grinding cup and 3x20 mm balls (Retsch centrifugal ball mill, S1). The ground product is dried in a desiccator for 24 hours.

### Adapted from U.S. Patent No. 5,827,502

**Table III**

| Example No. | Surfactant | Amount of Surfactant (% w/w) | Amount of ethanol (or water) (g) |
|---|---|---|---|
| 1 | Lecithin | 1.0 | 1.2 |
| 2 | " | 0.2 | 1.2 |
| 3 | Sodium Lauryl Sulphate | 1.0 | 1.0 (water) |
| 4 | " | 0.1 | 1.0 (water) |
| 5 | Span 80 | 1.0 | 1.2 |
| 6 | " | 0.1 | 1.2 |
| 7 | Span 85 | 1.0 | 1.2 |
| 8 | " | 0.1 | 1.2 |
| 9 | Pluronic F68 | 1.0 | 1.2 |
| 10 | " | 0.1 | 1.2 |
| 11 | Sodium Docusate | 1.0 | 1.2 |
| 12 | " | 0.1 | 1.2 |
| 13 | DOPE | 1.0 | 1.2 |
| 14 | " | 0.1 | 1.2 |
| 15 | α-Glyceryl Monolaurate | 0.2 | 3.2 |
| | Glyceryl Tripalmitate | 0.2 | |
| | Cholestrol | 0.2 | |
| | Cholestrol Acetate | 0.2 | |
| 16 | α-Glyceryl Monolaurate | 0.02 | 1.2 |
| | Glyceryl Tripalmitate | 0.02 | |
| | Cholestrol | 0.02 | |
| | Cholestrol Acetate | 0.02 | |
| | Cholestrol Benzoate | 0.02 | |
| 17 | Hexadecanedioic Acid | 0.0 | 1.2 |
| 18 | Linolenic Acid | 1.0 | 1.2 |

### EXAMPLES 19-22

The general procedure for Examples 1-18 is repeated except that the D-(+)-galactose is replaced by the carbohydrates listed in Table IV, in the amounts and using the quantities of water shown, and that the surfactant used is palmitic acid (0.2% w/w relative to the carbohydrate) dissolved in 96% ethanol.

### Adapted from U.S. Patent No. 5,827,502

**Table IV**

| Example No. | Microbubble-generating Carbohydrate | Amount of Carbohydrate (g) | Amount of water) (g) |
|---|---|---|---|
| 19 | Xylose (BDH) | 10.0 | 14.2 |
| 20 | Maltodextrin | 10.0 | 14.2 |
| 21 | Glycogen (Merck) | 5.0 | 17.2 |
| 22 | α-Cvclodextrin (Sigma) | 0.1 | 17.2 |

### 6.2. Patient Preparation

In the following examples, a patient undergoes embolization for uterine fibroid. One of ordinary skill in the relevant art would recognize that any drugs may be administered to a patient in preparation of an embolization procedure. As described in Worthington-Kirsch et al., 1998, Radiology 208:625-629, prior to embolization, a patient is treated with one dose of prophylactic antibiotics (cefazolin sodium or clindamycin phosphate administered intravenously) and undergoes a pelvic ultrasound examination (Model 128; Acuson, Mountain View, Calif.).

### 6.2.1. Embolization Procedure

The following method of embolization for uterine fibroid is used as described in Goodwin et al., 1997, Journal of Vascular and Interventional Radiology 8(4):517-526. By means of a femoral arterial approach, embolization is performed on a digital angiographic unit (Advantx; GE Medical Systems, Milwaukee, WI). A5-F, Levin-1 catheter (Cook, Bloomington, IN) or a 4-F, C1 Glidecath (Medi-tech, Boston Scientific, Watertown, MA) is used to catheterize the anterior division of the contralateral internal iliac artery. In patients with large uterine arteries, the same catheter is used to subselect and embolize the uterine artery. Tracker 18 microcatheter/Seeker wire combination (Target, Fremont, CA) is employed in a coaxial fashion to catheterize and embolize the uterine artery. Subselective catheterization is facilitated by the use of digital road mapping.

The Waltman loop technique is usually used to enter the ipsilateral uterine artery, and subselective catheterization and embolization are performed as described in Waltman et al., 1973, Radiology 109:732-734. Ultrasound modalities are used thereafter to follow the embolization as embolization composition is introduced into the patient. Alternatively, transcutaneous pathway is used and ultrasound is employed to position the catheter and follow the embolization procedure as embolization composition is introduced into the patient. Polyvinyl (PVA) particles (355-500 mm or 500-700mm) are utilized for embolization. An average of 500mg is used for bilateral embolization. The entire procedure takes an average of 75 minutes.

### 6.2.2. Sedation and Analgesia

General anesthesia or conscious sedation with midazolam and fentanyl is used as described in Worthington-Kirsch et al., 1998, Radiology 208:625-629. Immediately prior to embolization, 100 mg of lidocaine is injected directly into each uterine artery for periprocedural analgesia. A 60 mg intravenous or intramuscular dose of ketorolac tromethamine is given to a patient who is monitored for 6 hours after the procedure. Oral (codeine, oxycodone) and intravenous (morphine, hydromorphone) analgesics are given as needed.

### 6.2.3. Follow-up Ultrasound Examination

At approximately 3 months after embolization, ultrasound examination is performed on the patients as described in Goodwin et al., 1997, Journal of Vascular and Interventional Radiology 8(4):517-526.. The percent of reduction in uterine volume is calculated based on measurements of the uterine longitudinal, transverse, and anteroposterior axes.

### 6.2.4. In Vitro Echogenicity

The following example sets forth an exemplary protocol for determining echogenicity in vitro. The present example measures the acoustic effects at a level such that the products can be expected to be useful to be detected by ultrasound. However, one of ordinary skill in the relevant art will recognize that the techniques of this example can be utilized for determining desirable echogenicity in any appropriate embolic particles, agents or compositions of the present invention.

In vitro Echogenicity is determined by the methods as described in U.S. Patent 5,827,502 and U.S. Patent 5,795,562.

The embolic particles of the invention are redispersed in an aqueous solvent by shaking on a laboratory shaker for 12-16 hours. Alternatively, the embolic agent or composition of the invention is obtained by measuring the ultrasonic transmission through suspensions of different concentrations in an aqueous carrier liquid. Infusion of the solution in the measurement cell and the acoustic effects of the products are investigated by measuring the acoustic transmission through the samples using 3.5-5 MHZ broadband transducer in a pulse-reflection technique. The temperature in the measurement cell is stabilized to 37°C and circulation of the liquid is maintained by stirring at a constant rate. Results are normalized to measurements of a carrier reference sample. Measurements are performed along a dilution line where the starting suspension is successively diluted with the carrier liquid. Measurements are done until the signal was reduced to approximately 3-5 db/cm. At this acoustic level the products can be expected to be useful to be detected by ultrasound. Solid (as oppose to gas containing) particles of the same size and at the same dilutions should give an acoustic attenuation of less than 0.1db/cm.

### 6.2.5. In Vivo Characterization

The following example sets forth an exemplary protocol for in vivo characterization of the embolic particle, agent or composition of the invention. The present example utilizes rabbits as a tool to characterize the product of the invention. However, one of ordinary skill in the relevant art would recognize that the techniques of this example can be utilized for characterization of the product of the present invention in other animals.

Generally, embolic particles are characterized in vivo as described in U.S. Patent 5,795,562. Dry powder of embolic particles of the invention is redispersed in a sterile 0.9% (wt/wt) NaCl (aq) solution by shaking on a laboratory shaker for 12-16 hours. The dispersions are injected in chinchilla rabbits, and measured by using a doppler technique where an ultrasound probe is placed directly on a carotid artery and the inferior caval vein. The particle dispersions are injected in an ear vein. Signal height and duration are recorded. The obtained signal heights indicate an *in vivo* ultra sound contrast effect for the dispersions. The long duration of signal indicates that the *in vivo* stability is good.

## Claims

1. An embolic particle comprising one or more voids such that the embolic particle is visible under ultrasound, wherein the void contains a low pressure gas or a vacuum.

2. The embolic particle of claim 1 wherein the void contains one or more low pressure gases or microbubbles of low pressure gases.

3. The embolic particle of claim 2 wherein the gas or microbubbles of gas is selected from the group consisting of carbon dioxide, air, nitrogen, oxygen, nitrogen oxide, nitrous oxide, helium, argon, sulphur hexafluoride, methane, acetylene and carbon tetrafluoride.

4. The embolic particle of claim 1 further comprising a compatible carrier.

5. The embolic particle of anyone of claims 1 to 2, where the embolic particles are made of a material selected from the group consisting of silicone, polyester, vinyl polymer, fibrin, cellulosic, alginate, polyvinyl alcohol (PVA), gelatin, acrylate polymer, collagen, polyolefins, polyethers, polystyrene, polyurethane, ethylene vinyl alcohol, polyethylene polymer (PVP), polylactic acid (PLA), polymethylmethacrylate (PMMA), polyethylene glycol (PEG), and any copolymer of the foregoing.

6. The embolic particle of anyone of claims 1 to 5 for use in a method of treating a disease in a subject in need of such treatment comprises introducing the embolic particle to the site to be occluded.

7. The embolic particle of claim 6, wherein the disease is selected from the group consisting of solid tumor, cancer, urinary bulking, arteriovenous malformations, varicocele and pelvic congestion.

8. The embolic particle of claim 6, wherein the disease is uterine fibroid.

9. An embolization kit comprising the particle according to claim 2.

## Patentansprüche

1. Embolisierendes Partikel, umfassend einen oder mehrere Hohlräume, sodass das embolisierende Partikel unter Ultraschall sichtbar ist, wobei der Hohlraum ein Niederdruckgas oder ein Vakuum beinhaltet.

2. Embolisierendes Partikel nach Anspruch 1, wobei der Hohlraum ein oder mehrere Niederdruckgase oder Mikroblasen aus Niederdruckgasen beinhaltet.

3. Embolisierendes Partikel nach Anspruch 2, wobei das Gas oder die Mikroblasen aus Gas ausgewählt sind aus der Gruppe bestehend aus Kohlendioxid, Luft, Stickstoff, Sauerstoff, Stickoxid, Distickstoffoxid, Helium, Argon, Schwefelhexafluorid, Methan, Acetylen und Tetrafluormethan.

4. Embolisierendes Partikel nach Anspruch 1, weiter umfassend einen kompatiblen Träger.

5. Embolisierendes Partikel nach einem der Ansprüche 1 bis 2, wobei die embolisierenden Partikel hergestellt sind aus einem Material, ausgewählt aus der Gruppe bestehend aus Silikon, Polyester, Vinylpolymer, Fibrin, Zellulose, Alginat, Polyvinylalkohol (PVA), Gelatine, Polyacrylat, Kollagen, Polyolefine, Polyether, Polystyren, Polyurethan, Ethylenvinylalkohol, Polyethylenpolymer (PVP), Polylactide (PLA), Polymethylmethacrylat (PMMA), Polyethylenglykol, und jegliches Copolymer der Vorangehenden.

6. Embolisierendes Partikel nach einem der Ansprüche 1 bis 5 zur Benutzung in einem Verfahren zur Behandlung einer Erkrankung eines Patienten, der eine solche Behandlung benötigt, umfassend Zuführen des embolisierenden Partikels zu der zu verschließenden Stelle.

7. Embolisierendes Partikel nach Anspruch 6, wobei die Erkrankung ausgewählt ist aus der Gruppe, bestehend aus solider Tumor, Krebs, Urinverdickung, arteriovenöse Malformationen, Varikozele und Varikosis pelvis.

8. Embolisierendes Partikel nach Anspruch 6, wobei die Erkrankung ein Uterusmyom ist.

9. Embolisierungskit, umfassend das Partikel nach Anspruch 2.

## Revendications

1. Particule embolique comprenant une ou plusieurs cavités de telle sorte que la particule embolique est visible sous ultrasons, où la cavité contient un gaz sous basse pression ou sous vide.

2. Particule embolique selon la revendication 1, dans laquelle la cavité contient un ou plusieurs gaz sous basse pression ou des microbulles de gaz sous basse pression.

3. Particule embolique selon la revendication 2, dans laquelle le gaz ou les microbulles de gaz est choisi dans le groupe constitué de dioxyde de carbone, air, azote, oxygène, oxyde azote, oxyde nitreux, hélium, argon, hexafluorure de soufre, méthane, acétylène et tétrafluorure de carbone.

4. Particule embolique selon la revendication 1, comprenant en outre un support compatible.

5. Particule embolique selon l'une quelconque des revendications 1 et 2, dans laquelle les particules emboliques sont constituées d'un matériau choisi dans le groupe constitué de silicone, un poly(ester), polymère de vinyle, fibrine, cellulosique, alginate, poly(alcool vinylique) (PVA), gélatine, polymère d'acrylate, collagène, poly(oléfines), poly(éthers), poly(styréne), poly(uréthane), éthylène-alcool vinylique, polymère de poly(éthyléne) (PVP), poly(acide lactique) (PLA), poly(méthacrylate de méthyle) (PMMA), poly(éthyléne glycol) (PEG), et tout copolymère des matériaux précédents.

6. Particule embolique selon l'une quelconque des revendications 1 à 5, à utiliser dans un procédé de traitement d'une maladie chez un sujet en besoin d'un tel traitement comprenant l'introduction de la particule embolique dans le site à occlure.

7. Particule embolique selon la revendication 6, dans laquelle la maladie est choisie dans le groupe constitué d'une tumeur solide, cancer, encombrement urinaire, malformations artério-veineuses, varicocèle et congestion pelvienne.

8. Particule embolique selon la revendication 6, dans laquelle la maladie est un fibrome utérin.

9. Matériel d'embolisation comprenant la particule selon la revendication 2.
